# EUROPEAN PATENT SPECIFICATION

(11) **EP 1 449 519 B1**
(45) Date of publication and mention of the grant of the patent: **09.07.2008**
(21) Application number: 04250815.0
(22) Date of filing: 16.02.2004
(51) Int. Cl.: A61Q 19/10, A61Q 19/00, A61Q 15/00

(54) **Liquid composition containing a guanidine amide group derivative and/or a salt thereof**
Eine flussige Zubereitung enthaltend ein Amide substuiertes Guanidine derivate oder Salze davon
Composition liquide comprenant un guanidine substitué avec un groupement amide et /ou c'est dérivés

(30) Priority: 20.02.2003 JP 2003042576
(43) Date of publication of application: 25.08.2004
(73) Proprietor: Ajinomoto Co., Inc., Tokyo 104-8315 (JP)
(72) Inventor: Kanatani, Eizo, Kawasaki-shi, Kanagawa-ken 210-8681 (JP); Yumioka, Ryosuke, Chuo-ku, Tokyo 104-8315 (JP); Miyazawa, Kiyoshi, Mitoyo-gun, Kagawa-ken 769-1602 (JP); Ikeda, Hiroko, Shizuoka-ken 436-0033 (JP)
(74) Representative: Nicholls, Kathryn Margaret

(56) References cited:
- EP-A- 1 285 579
- DE-A- 4 425 080
- US-A- 4 975 275

## Description

The present invention relates to a composition containing a specific amide group-containing guanidine derivative or/and a salt thereof. More specifically, the invention relates to a method for suppressing the generation of the precipitate of an amide group-containing guanidine derivative or/and a salt thereof (component A), including a step of blending at least one (component B) selected from the group consisting of specific inorganic salts, organic acid salts, nonionic surfactants and ampholytic surfactants to the amide group-containing guanidine derivative or/and a salt thereof; and a liquid composition with improved storage stability, which contains the amide group-containing guanidine derivative or/and a salt thereof. The liquid composition canbe applied to awidevarietyof products such as pharmaceutical fluids for wet tissue, cosmetic lotions, mouthwash agents, lotions, bathing agents and deodorants.

In the surge of increasing social concerns toward safety profiles and environmental awareness, various amino acid derivatives are now increasingly researched actively. Naturally occurring amino acid derivatives, N-long chain acyl basic amino acid derivatives and salts thereof have been known traditionally as bactericidal washing agents, which are cationic activators like quaternary ammonium salts (JP-B-51-5413) . For example, N-cocoyl-L-arginine ethyl ester-DL-pyrrolidone carboxylate salt as one of them is commercially available under the trade name "CAE" and has a great surface-acting activity and a great safety profile as well as a high preservative activity or a bactericidal activity.

However, N-long chain acyl basic amino acid derivatives are generally used in salt forms and are therefore soluble in water. Accordingly, the ester groups in the molecules of the salts of the derivatives are sometimes hydrolyzed.

Meanwhile, amino group-containing guanidine derivatives or salts thereof having the structure of N-long chain acyl basic amino acid derivatives, from which the ester group is preliminarily removed, has great stability against hydrolysis, as expected on the basis of the structure. As described in JP-A-2-243614 (Patent document 1), JP-A-4-49221 (Patent document 2) and JP-A-4-49222 (Patent document 3), additionally, the derivatives or salts thereof have a great hair-adsorbing property and are therefore used as surfactants giving softness, emollience and great finish.

In case that the derivatives or salts thereof are used as external agents for skin, it has been known that external agents for skin having a great emollient effect, marked spreadability during use and highly smooth touch without any stickiness during use can be provided, as disclosed in JP-A-6-321727 (Patent document 4)

The inventors prepared pharmaceutical fluids for wet tissue, cosmetic lotions and the like (liquid compositions) using the amide group-containing guanidine derivative represented by the general formula (1) or a salt thereof as a surfactant, a bactericidal agent and a preservative. However, precipitates emerged in these liquid compositions, just when the liquid compositions were diluted or were left to stand over time. The inventors found that these compositions had a problem of storage stability

Such phenomenon has never been confirmed in emulsified opaque states of hair cosmetics such as conditioner. The problem has been observed more clearly in liquid compositions semi-transparent to transparent.

The inventors analyzed the generated precipitates. Consequently, the inventors confirmed that the precipitates were fatty acids secondarily produced during the synthetic reaction of the amide group-containing guanidine derivative represented by the general formula (1) and salts of the amide group-containing guanidine derivative.

As described above, the amide group-containing guanidine derivative represented by the general formula (1) and salts thereof are useful compounds as surfactants, touch-improving agents, preservatives or bactericides. However, the product values thereof are deteriorated when liquid compositions containing the derivative or salts thereof happen to form precipitates. Otherwise, the touch thereof during use is deteriorated when used on skin. From the standpoint of the process control of the industrial preparation of an aqueous solution of the amide group-containing guanidine derivative and salts thereof, the dissolution of the amide group-containing guanidine derivative and salts thereof cannot be visually confirmed in a simple manner, disadvantageously.

In such circumstances it is desirable that embodiments of the invention provide a method for suppressing the generation of the precipitate of the amide group-containing guanidine derivative represented by the general formula (1) or/and a salt thereof in a liquid composition containing the derivative or/and a salt thereof, and also provides such liquid composition with improved stability.

In the first aspect, the present invention relates to the use of at least one component B, selected from an inorganic salt, an organic acid salt, a nonionic surfactant and an ampholytic surfactant for suppressing the generation of precipitate of a component A in a liquid composition containing component A and component B in a weight ratio of 1/0.5 to 1/20wherein component A is an amide group-containing guanidine derivative represented by the following general formula (1): 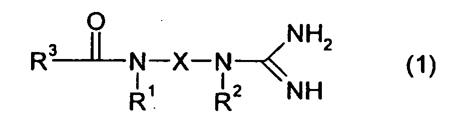 (in the formula, R¹ and R² are independently hydrogen atom or a linear or branched alkyl group or alkenyl group with one to 4 carbon atoms and may be the same or different; R³ represents a linear or branched alkyl group or alkenyl group with one to 22 carbon atoms; and X represents a linear or branched alkylene group or alkenylene group with one to 10 carbon atoms) or/and a salt thereof.

In a second aspect, the present invention provides a liquid composition containing an amide group-containing guanidine derivative represented by the following general formula (1): 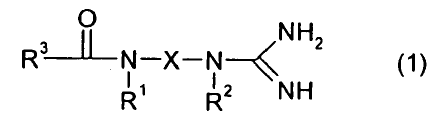 (in the formula, R¹ and R² are independently hydrogen atom or a linear or branched alkyl group or alkenyl group with one to 4 carbon atoms and may be the same or different; R³ represents a linear or branched alkyl group or alkenyl group with one to 22 carbon atoms; and X represents a linear or branched alkylene group or alkenylene group with one to 10 carbon atoms) or/and a salt thereof (component A) and at least one component B selected from sodium chloride, potassium chloride, sodium glutamate (monohydrate), sodium aspartate, potassium aspartate, sodium lactate, potassium lactate, sodium malate, potassium malate and potassium acetate in which the blend ratio of component A/component B, by weight, is from 1/0.5 to 1/20.

Embodiments of the invention are now described in detail hereinbelow.

The amide group-containing guanidine derivative represented by the following general formula (1) and a salt thereof (component A) in accordance with the invention is first described in detail hereinbelow.

The amide group-containing guanidine derivative in accordance with the invention is represented by the following general formula (1). 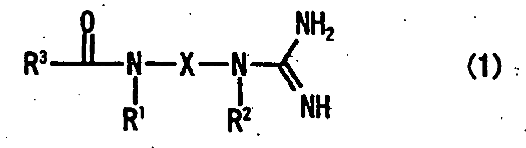 (In the formula, R¹ and R² are independently hydrogen atom or a linear or branched alkyl group or alkenyl group with one to 4 carbon atoms and may be the same or different; R³ represents a linear or branched alkyl group or alkenyl group with one tao 22 carbon atoms; and X represents a linear or branched alkylene group or alkenylene group with one to 10 carbon atoms).

The long-chain acyl group (R³CO) in the amide group-containing guanidine derivative represented by the general formula (1) in accordance with the invention maybe linear or branched and saturated or unsaturated and has one to 22 carbon atoms and includes acyl groups prepared from for example acetic acid, propionic acid, capric acid, lauric acid, myristic acid, palmitic acid, stearic acid, behenic acid, linoleic acid, linolenic acid, oleic acid, isostearic acid, 2-ethylhexanoic acid, coconut oil fatty acid, fatty acids in the fats and oils in beef tallow, and fatty acids in the fats and oils in hardened beef tallow. Preferably, the acyl group includes caproyl group, lauroyl group, myristyl group, palmitoyl group, stearoyl group, behenoyl group, cocoyl group and acyl groups of fatty acids in hardened beef tallow. More preferably, the acyl group includes relatively inexpensively available lauroyl group, myristyl group, palmitoyl group and stearoyl group. Among them, most preferably, the acyl group is lauroyl group with the highest antimicrobial activity. Additionally, the acyl group includes not only an acyl group of a single chain length but also a mixture of acyl groups with different chain lengths.

Further, X is a branched or linear alkylene group or alkenylene group with one to 10 carbon atoms, preferably 2 to 6 carbon atoms and includes for example ethylene, propylene, tetramethylene, pentamethylene and hexamethylene, particularly preferably tetramethylene group in 4-aminobutylguanidine produced from a naturally occurring amino acid arginine.

Herein, the amide group-containing guanidine derivative of the invention is generally used in salt forms. Specifically, the amide group-containing guanidine derivative can be used in the forms of inorganic acid salts such as hydrochloride salt, organic acid salts such as glycolate salt, acetate salt, lactate salt and acidic amino acid salts. One of an amido group-containing guanidine derivative and a salt thereof can be blended singly in the liquid composition of the invention or both thereof can be concurrently blended therein.

The amide group-containing guanidine derivative represented by the following general formula (1) or a salt thereof in accordance with the invention can be produced for example by treating monoamide amine prepared from diamine at a heating process under reduced pressure or a nitrogen bubbling process under heating and then storing the resulting product in carbon dioxide-free atmosphere, and guanidylating thereafter the product with cyanamide, S-methylisothiourea or the like, and removing contaminating impurities by measures such as crystallization, as disclosed in JP-A-6-312972.

The amide group-containing guanidine derivative or a salt thereof in accordance with the invention can additionally be produced by reaction of an amino group-containing guanidine derivative represented by the following general formula (2) with a fatty acid halide or with a fatty acid ester. However, it is difficult to sufficiently remove fatty acids secondarily produced during such synthetic reactions. 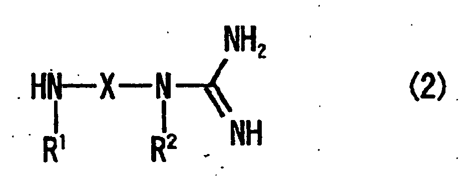 (In the formula, R¹ and R² are independently hydrogen atom or a linear or branched alkyl group or alkenyl group with one to 4 carbon atoms and may be the same or different; and X represents a linear or branched alkylene group or alkenylene group with one to 10 carbon atoms.)

The inorganic salts, organic acid salts, nonionic surfactants and ampholytic surfactants (component B) with a great activity to suppress the generation of the precipitate of the amide group-containing guanidine derivative represented by the general formula (1) and a salt thereof in a liquid composition containing the derivative and a salt thereof is now described in detail hereinbelow.

Any inorganic salts, organic acid salts, nonionic surfactants and ampholytic surfactants (generally referred to as precipitation-suppressing agent hereinbelow) with an action to suppress the generation of the precipitate of the amide group-containing guanidine derivative represented by the general formula (1) and a salt thereof are used in accordance with the invention with no specific limitation. As such, generally, an appropriate one from those for use in cosmetics can be selected and used, depending on the case. A person skilled in the art can readily determine whether or not the selected one is appropriate as the precipitation-suppressing agent, with reference to the following Examples.

Examples of the inorganic salt include sodium chloride and potassium chloride.

Examples of the organic acid salt include sodium glutamate (monohydrate), potassium glutamate, sodium aspartate, potassium aspartate, sodium lactate, potassium lactate, sodium malate, potassium malate, sodium acetate, ammonium acetate, potassium acetate, sodium salicylate, sodium benzoate, sodium sorbate, and disodium edetate.

Among these organic acid salts, sodium glutamate (monohydrate), sodium aspartate, potassium aspartate, sodium lactate, potassium lactate,sodium malate and potassium acetate are preferable. Among them, sodium lactate and sodium glutamate are more preferable.

The nonionic surfactant includes for example propylene glycol fatty acid ester, glycerin fatty acid ester, polyoxyethylene glycerin fatty acid ester, polyglycerin fatty acid ester, sorbitan fatty acid ester, polyoxyethylene sorbitan fatty acid ester, polyoxyethylene sorbit fatty acid ester, polyoxyethylene alkylphenylformaldehyde condensates, polyoxyethylene castor oil, polyoxyethylene hardened castor oil, polyoxyethylene sterol and derivatives thereof, polyethylene glycol fatty acid ester, polyoxyethylene alkyl ether, polyoxyethylene polyoxypropylene alkyl ether, polyoxyethylene alkylphenyl ether, polyoxyethylene lanoline and derivatives thereof, polyoxyethylene bee wax derivatives, polyoxyethylene alkylamine, polyoxyethylene fatty acid amide, alkanol amide, sugar esters, polyoxyethylene hardened castor oil pyroglutamate ester, and polyoxyethylene glyceryl pyroglutamate ester.

Among these nonionic surfactants, sorbitan fatty acid ester, polyoxyethylene sorbit fatty acid ester, polyoxyethylene hardened castor oil, polyethylene glycol fatty acid ester, polyoxyethylene hardened castor oil pyroglutamate ester, and polyoxyethylene glyceryl pyroglutamate ester are preferable. Among them, sorbitan fatty acid ester, polyoxyethylene sorbit fatty acid ester, polyoxyethylene hardened castor oil pyroglutamate ester, and polyoxyethylene glyceryl pyroglutamate ester are more preferable.

Examples of the ampholytic surfactant include carbobetaine-type ampholytic surfactants, sulfobetaine-type ampholytic surfactants, hydroxysulfobetaine-type ampholytic surfactants, amide sulfobetaine-type ampholytic surfactants, phosphobetaine-type ampholytic surfactants, imidazoline-type ampholytic surfactants and lecithin derivatives Among these ampholytic surfactants, carbobetaine-type ampholytic surfactants, sulfobetaine-type ampholytic surfactants and hydroxysulfobetaine-type ampholytic surfactants are preferable. Among them, further, carbobetaine-type ampholytic surfactants are more preferable.

The amide group-containing guanidine derivative represented by the general formula (1) or/and a salt thereof (component A) contained in the liquid composition of the invention can generally be within a range of for example 0.001 to 5.0 % by weight, preferably 0.01 to 2.0 % by weight, more preferably 0.05 to 1.0 % by weight of the total weight of the liquid composition

The inorganic salt,organic acid salt,non ionic surfactant or ampholytic surfactant (component B) as a precipitation-suppressing agent can be blended at any amount within a range with no deterioration of the advantages of the invention, with no other specific limitation. Generally, the precipitation-suppressing agent can be used within a range of for example 0.01 to 10.0 % by weight, preferably 0.1 to 5.0 % by weight, more preferably 0.2 to 2.0 % by weight of the tonal weight of the liquid composition. It is needless to say that two or more of the inorganic salt, organic acid salt, nonionic surfactant and ampholytic surfactant can be used in combination as component B. In case of such use in combination, the total amount thereof can be within the range described above.

Additionally, the blend ratio of the components A and B can be appropriately determined readily, depending on the intended product (liquid composition). Generally, the components A and B are used within a range of the component A/component B (in weight ratio) = 1/0.5 to 1/20. When the blend ratio is more than 1/0.5, the effect on suppressing precipitation is insufficient. When the blend ratio is less than 1/20, the resulting precipitation-suppressing agent at an amount more than necessary works as impurities to reduce the product value. Additionally, the precipitation-suppressing agent may sometimes separate the component A in oily matter. From the standpoint of retaining the sustainable effect on suppressing precipitation, preferably, the component A/component B (in weight ratio) = 1/1 to 1/10. More preferably, the component A/component B (in weight ratio) = 1/2 to 1/5.

As optional components, various additives for general use can be added within a range without any deterioration of the advantages of the invention, to the liquid composition of the invention. Examples thereof include surfactants, emollients, silicone compounds, polymer substances (polymer compounds), alcohols, ultraviolet absorbents, dyes, pigments, vitamin, antioxidants, metal ion sealing agents, preservatives, bactericides, pH adjusters, pearling agents, nucleic acid, enzymes, and raw materials such as naturally-occurring extracts according to the Raw Material Standards for Cosmetics, the Blend Components Standards for Individual Cosmetic Types, the Raw Material Standards for Pharmaceutical Products, the Japanese Pharmacopoeia, and the Official Food Additives Standards.

The liquid composition of the invention can be produced by general methods in this art, such as a process of dissolving the amide group-containing guanidine derivative represented by the general formula (1) or/and a salt thereof (component A) and at least one of the precipitation-suppressing agents such as the inorganic salt, the organic acid salt, the nonionic surfactant and the ampholytic surfactant (component B) in water. The liquid composition of the invention can be applied to a wide variety of products such as pharmaceutical fluids for impregnating supporting base materials, external agents for skin, cosmetic lotions, mouthwash agents, lotions, bathing agents, and deodorants.

Finally, the mode of distributing the liquid composition of the invention is now described. As described above, the liquid composition of the invention is produced by dissolving various additives for routine use, if necessary, in addition to the components A and B, in water or the like. The liquid composition may be distributed in a so-called premix form before dissolving in water or the like, without being prepared as such final form. A person having purchased the premix can readily dissolve the premix in water or the like to prepare the final form of the liquid composition. Thus, such premix is also included within the scope of the invention. It is needless to say that the ratio of the components A and B contained in the premix should be identical to those described above.

Embodiments of the invention are now described in the following Examples.

### <Comparative Examples 1 through 3 and Examples 1 through 5: Selection of precipitation-suppressing agents>

A substance described below in Table 1 was added to an aqueous 0.1 % by weight solution of lauramide butylguanidine hydrochloride salt to 0.2 % by weight, for testing the storage stability at 25°C. The state of the resulting solution was confirmed visually, immediately after dissolution, one day later and 7 days later. The case of transparent dissolution was marked with O, while the case with precipitates was marked with x. The results are shown below in Table 1.

**Table 1**

| Examples | Additives | 25°C | | |
|---|---|---|---|---|
| | | immediately after dissolution | one day later | 7 days later |
| Comparative Example 1 | no additive | × | × | × |
| Comparative Example 2 | sodium sulfate | × | × | × |
| Comparative Example 3 | sodium dihydrogen phosphate | × | × | × |
| Example 1 | sodium chloride | ○ | ○ | ○ |
| Example 2 | potassium chloride | ○ | ○ | ○ |
| Example 3 | sodium lactate | ○ | ○ | ○ |
| Example 4 | sodium glutamate monohydrate | ○ | ○ | ○ |
| Example 5 | POE (40) hardened castor oil pyroglutamate isostearate diester | ○ | ○ | ○ |

| | | | | |
|---|---|---|---|---|
| Assessment dissolved transparently: ○; precipitated: ×. | | | | |

The results in Table 1 indicate that additives (precipitation-suppressing agents) with an effective activity to suppress the generation of the precipitate of the amide group-containing guanidine derivative used herein could be selected.

### <Comparative Example 1 and Examples 6 through 15: effective concentration of precipitation-suppressing agents>

Sodium chloride or potassium chloride was added to an aqueous 0.1 % by weight solution of lauramide butylguanidine hydrochloride salt to various concentrations, for testing the storage stability at 25°C. The states of the resulting solutions were confirmed visually, immediately after dissolution, one day later and 7 days later. The case of transparent dissolution was marked with ○, while the case with slight precipitates was marked with Δ and the case with precipitates was marked with ×. The results are shown below in Tables 2 and 3.

**Table 2**

| Examples | Amount of sodium chloride added | 25°C | | |
|---|---|---|---|---|
| | | immediately | one day later | 7 days later |
| Comparative Example 1 | not added | × | × | × |
| Example 8 | 0.05% | ○ | Δ | Δ |
| Example 7 | 0.1% | ○ | ○ | ○ |
| Example 8 | 0.2% | ○ | ○ | ○ |
| Example 9 | 0.5% | ○ | ○ | ○ |
| Example 10 | 1.0% | ○ | ○ | ○ |

| | | | | |
|---|---|---|---|---|
| Assessment dissolved transparently: ○; slightly precipitated: Δ; precipitated: ×. | | | | |

**Table 3**

| Examples | Amount of potassium chloride added | 25°C | | |
|---|---|---|---|---|
| | | immediately after dissolution | one day later | 7 days later |
| Comparative Example 1 | not added | × | × | × |
| Example 11 | 0.05 % | Δ | Δ | Δ |
| Example 12 | 0.1% | ○ | ○ | ○ |
| Example 13 | 0.2% | ○ | ○ | ○ |
| Example 14 | 0.5% | ○ | ○ | ○ |
| Example 15 | 1.0% | ○ | ○ | ○ |

| | | | | |
|---|---|---|---|---|
| Assessment: dissolved transparently: ○; slightly precipitated: Δ; precipitated: ×. | | | | |

As clearly shown in the results in Tables 2 and 3, it was confirmed that the addition of sodium chloride or potassium chloride improved the storage stability. In the Examples, great storage stability one day later and thereafter couldbe confirmed at 0.1 % by weight or more of sodium chloride. It is needless to say that the type and concentration of the additive can be modified, depending on the term and conditions required for the storage stability, in light of the product and form of the liquid composition.

### <Examples 16 through 19: preparation of cosmetic lotions>

Cosmetic lotions of the following compositions (expressed in % by weight; total = 100 %) shown below in Table 4 were prepared. The resulting cosmetic lotions had great storage stability and high emollience with no stickiness, giving great touch during use.

**Table 4**

| | Example 16 | Example 17 | Example 18 | Example 19 |
|---|---|---|---|---|
| Component A | | | | |
| Lauramide butylguanidine hydrochloride salt | 0.1 | | 0.1 | |
| Lauramide butylguanidine lactate salt | | 0.2 | | 0.2 |
| Component B | | | | |
| Sodium lactate | 0.4 | | 0.2 | |
| Sodium chloride | | 0.2 | | |
| Hardened castor oil pyroglutamate isostearate diester | | | | 0.2 |
| Other components | | | | |
| Glycerin | 6 | 6 | 6 | 6 |
| Butylene glycol | 2 | 2 | 2 | 2 |
| Ethyl alcohol | 2 | 2 | 2 | 2 |
| German chamomile extract | 0.5 | 0.5 | 0.5 | 0.5 |
| Butyl paraben | 0.1 | 0.1 | 0.1 | 0.1 |
| Methyl paraben | 0.1 | | | 0.1 |
| Cetylpyridinium chloride | | 0.1 | 0.1 | |
| Fragrance | 0.1 | 0.1 | 0.1 | 0.1 |
| Distilled water | qs. | qs. | qs. | qs. |

### <Examples 20 through 21: preparation of mouthwash agents>

Mouthwash agents of the following compositions (expressed in % by weight; total = 100%)shown below in Table 5 were prepared. The resulting mouthwash agents had great storage stability and high bactericidal effects, giving great feeling during use.

**Table 5**

| | Example 20 | Example 21 |
|---|---|---|
| Component A | | |
| Lauramide butylguanidine hydrochloride salt | 0.1 | |
| Lauramide butylguanidine acetate salt | | 0.2 |
| Component B | | |
| Sodium chloride | | 0.4 |
| Sodium glutamate monohydrate | 0.2 | |
| Other components | | |
| Eucalyptol | 0.1 | 0.1 |
| Thymol | 0.06 | 0.06 |
| Menthol | - 0.04 | 0.04 |
| Poloxamer 407 | 0.2 | 02 |
| Methyl paraben | 0.1 | 0.1 |
| Glycerin | 0.5 | 0.5 |
| Propylene glycol | 2.5 | 25 |
| Distilled water | qs. | qs. |

### <Examples 22 through 25: preparation of wet tissues>

Pharmaceutical fluids of the following compositions (expressed in % by weight; total = 100 %) shown below in Table 6 were prepared. The resulting pharmaceutical fluids were used to impregnate a non-woven fabric of rayon and PET (non-woven fabric 1), a non-woven fabric of rayon and PP/PET (non-woven fabric 2) and a non-woven fabric of pulp (non-woven fabric 3) at a ratio of 2.5 to the individual weights thereof to prepare wet tissues. These wet tissues had great temperature stability and good antimicrobial anti-fungal property, also giving great touch during use with enriched emollient effect.

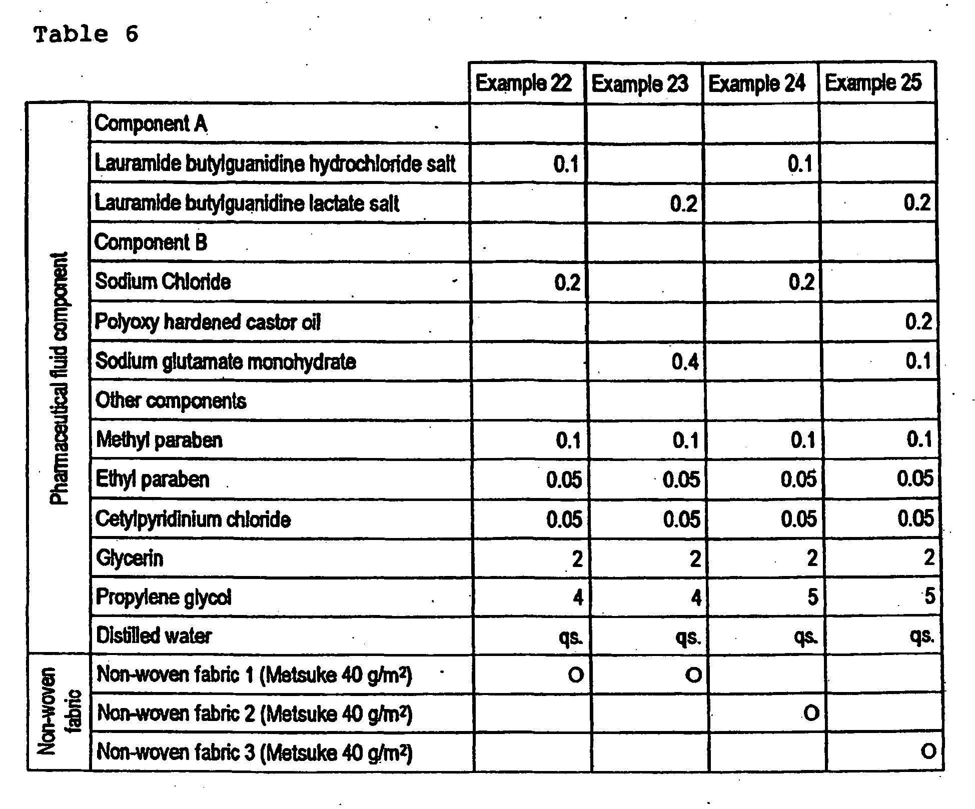

In accordance with embodiments of the invention, the generation of precipitates in the liquid composition containing the amide group-containing guanidine derivative represented by the general formula (1) or/and a salt thereof can be readily suppressed, so that the storage stability of such liquid composition can be enhanced. Consequently, the product value thereof can be readily improved.

## Claims

1. Use of at least one component B, selected from an inorganic salt, an organic acid salt, a nonionic surfactant and an ampholytic surfactant for suppressing the generation of precipitate of a component A in a liquid composition containing component A and component B in a weight ratio of 1/0.5 to 1/20, wherein component A is an amide group-containing guanidine derivative represented by the following general formula (1): 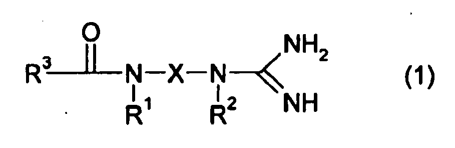 (in the formula, R¹ and R² are independently hydrogen atom or a linear or branched alkyl group or alkenyl group with one to 4 carbon atoms and may be the same or different; R³ represents a linear or branched alkyl group or alkenyl group with one to 22 carbon atoms; and X represents a linear or branched alkylene group or alkenylene group with one to 10 carbon atoms) or/and a salt thereof.

2. A use according to claim 1, wherein the inorganic salt is selected from the group consisting of sodium chloride and potassium chloride.

3. A use according to claim 1, wherein the organic salt is selected from the group consisting of sodium glutamate (monohydrate), sodium aspartate, potassium aspartate, sodium lactate, potassium lactate, sodium malate, potassium malate and potassium acetate.

4. A use according to claim 1, wherein the nonionic surfactant is selected from the group consisting of sorbitan fatty acid ester, polyoxyethylene sorbit fatty acid ester, polyoxyethylene hardened castor oil, polyethylene glycol fatty acid ester, polyoxyethylene hardened castor oil pyroglutamate ester and polyoxyethylene glyceryl pyroglutamate ester.

5. A use according to claim 1, wherein the ampholytic surfactant is selected from the group consisting of carbobetaine-type ampholytic surfactants, sulfobetaine-type ampholytic surfactants and hydroxysulfobetaine-type ampholytic surfactants

6. A use according to any one of claims 1 to 5, wherein R³ in the general formula (1) is a linear alkyl group with 11 carbon atoms.

7. A use according to any one of claims 1 to 5, wherein R¹ and R² in the general formula (1) are individually hydrogen atom; R³ is a linear alkyl group with 11 carbon atoms; and X is a linear alkyl group with 4 carbon atoms.

8. A liquid composition containing an amide group-containing guanidine derivative represented by the following general formula (1): 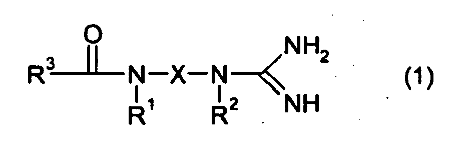 (in the formula, R¹ and R² are independently hydrogen atom or a linear or branched alkyl group or alkenyl group with one to 4 carbon atoms and may be the same or different; R³ represents a linear or branched alkyl group or alkenyl group with one to 22 carbon atoms; and X represents a linear or branched alkylene group or alkenylene group with one to 10 carbon atoms) or/and a salt thereof (component A) and at least one component B selected from sodium chloride, potassium chloride, sodium glutamate (monohydrate), sodium aspartate, potassium aspartate, sodium lactate, potassium lactate, sodium malate, potassium malate and potassium acetate in which the blend ratio of component A/component B, by weight, is from 1/0.5 to 1/20.

9. A liquid composition according to claim 8, wherein R³ in the general formula (1) is a linear alkyl group with 11 carbon atoms.

10. A liquid composition according to claim 8, wherein R¹ and R² in the general formula (1) are individually hydrogen atom; R³ is a linear alkyl group with 11 carbon atoms; and X is a linear alkyl group with 4 carbon atoms.

11. An impregnated supporting base material such as wet tissue, as prepared by impregnation with a liquid composition according to any one of claims 8 to 10.

## Patentansprüche

1. Verwendung mindestens einer Komponente B, ausgewählt unter einem anorganischen Salz, einem Salz einer organischen Säure, einem nicht ionischen oberflächenaktiven Mittel und einem ampholytischen oberflächenaktiven Mittel, zur Unterdrückung der Erzeugung eines Niederschlags einer Komponente A in einer flüssigen Zusammensetzung, die Komponente A und Komponente B in einem Gewichtsverhältnis von 1/0,5 bis 1/20 enthält, wobei Komponente A ein Amidgruppen enthaltendes Guanidinderivat der folgenden allgemeinen Formel (1): 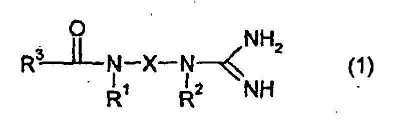 (in der Formel sind R¹ und R² unabhängig voneinander ein Wasserstoffatom oder eine lineare oder verzweigte Alkylgruppe oder Alkenylgruppe mit 1 bis 4 Kohlenstoffatomen und können gleich oder verschieden sein; R³ stellt eine lineare oder verzweigte Alkylgruppe oder Alkenylgruppe mit 1 bis 22 Kohlenstoffatomen dar; und X stellt eine lineare oder verzweigte Alkylengruppe oder Alkenylengruppe mit 1 bis 10 Kohlenstoffatomen dar) und/oder ein Salz davon ist.

2. Verwendung nach Anspruch 1, wobei das anorganische Salz aus der Gruppe ausgewählt ist, die aus Natriumchlorid und Kaliumchlorid besteht.

3. Verwendung nach Anspruch 1, wobei das organische Salz aus der Gruppe ausgewählt ist, das aus Natriumglutamat (Monohydrat), Natriumaspartat, Kaliumaspartat, Natriumlactat, Kaliumlactat, Natriummalat, Kaliummalat und Kaliumacetat besteht.

4. Verwendung nach Anspruch 1, wobei das nicht ionische oberflächenaktive Mittel aus der Gruppe ausgewählt ist, die aus Sorbitanfettsäureester, Polyoxyethylensorbitfettsäureester, Polyoxyethylen-gehärtetes Castor-Öl, Polyethylenglycolfettsäureester, Pyrroglutamatester von Polyoxyethylen-gehärtetem Castor-Öl und Polyoxyethylenglycerylpyrroglutamatester besteht.

5. Verwendung nach Anspruch 1, wobei das ampholytische oberflächenaktive Mittel aus der Gruppe ausgewählt ist, die aus ampholytischen oberflächenaktiven Mitteln vom Carbobetaintyp, ampholytischen oberflächenaktiven Mitteln von Sulfobetaintyp und ampholytischen oberflächenaktiven Mitteln vom Hydroxysulfobetaintyp besteht.

6. Verwendung nach einem der Ansprüche 1 bis 5, wobei R³ in der allgemeinen Formel (1) eine lineare Alkylgruppe mit 11 Kohlenstoffatomen ist.

7. Verwendung nach einem der Ansprüche 1 bis 5, wobei R¹ und R² in der allgemeinen Formel (1) unabhängig voneinander ein Wasserstoffatom darstellen; R³ eine lineare Alkylgruppe mit 11 Kohlenstoffatomen ist; und X eine lineare Alkylgruppe mit 4 Kohlenstoffatomen ist.

8. Flüssige Zusammensetzung, die ein Amidgruppen-enthaltendes Guanidinderivat der folgenden allgemeinen Formel (1): 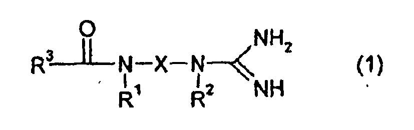 (in der Formel sind R¹ und R² unabhängig voneinander ein Wasserstoffatom oder eine lineare oder verzweigte Alkylgruppe oder Alkenylgruppe mit 1 bis 4 Kohlenstoffatomen und können gleich oder verschieden sein; R³ stellt eine lineare oder verzweigte Alkylgruppe oder Alkenylgruppe mit 1 bis 22 Kohlenstoffatomen dar; und X stellt eine lineare oder verzweigte Alkylengruppe oder Alkenylengruppe mit 1 bis 10 Kohlenstoffatomen dar) und/oder ein Salz davon (Komponente A) und mindestens eine Komponente B enthält, die unter Natriumchlorid, Kaliumchlorid, Natriumglutamat (Monohydrat), Natriumaspartat, Kaliumaspartat, Natriumlactat, Kaliumlactat, Natriummalat, Kaliummalat und Kaliumacetat ausgewählt ist, wobei das Mischungsverhältnis Komponente A/Komponente B 1/0,5 bis 1/20 als Gewichtsverhältnis ist.

9. Flüssige Zusammensetzung nach Anspruch 8, wobei R³ in der allgemeinen Formel (1) eine lineare Alkylgruppe mit 11 Kohlenstoffatomen ist.

10. Flüssige Zusammensetzung nach Anspruch 8, wobei R¹ und R² in der allgemeinen Formel (1) unabhängig voneinander ein Wasserstoffatom sind; R³ eine lineare Alkylgruppe mit 11 Kohlenstoffatomen ist; und X eine lineare Alkylgruppe mit 4 Kohlenstoffatomen ist.

11. Imprägniertes Trägermaterial, beispielsweise ein feuchtes Gewebe, hergestellt durch Imprägnieren mit einer flüssigen Zusammensetzung nach einem der Ansprüche 8 bis 10.

## Revendications

1. Utilisation d'au moins un composant B, choisi parmi un sel inorganique, un sel d'acide organique, un agent tensioactif non ionique et un agent tensioactif ampholytique pour supprimer la génération de précipité d'un composant A dans une composition liquide contenant un composant A et un composant B dans un rapport en poids de 1/0,5 à 1/20, dans laquelle le composant A est un dérivé guanidine contenant un groupe amide représenté par la formule générale (1) suivante: 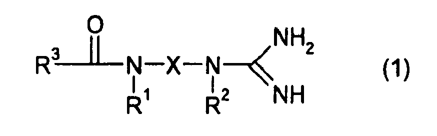 (dans la formule, R¹ et R² représentent indépendamment un atome d'hydrogène ou un groupe alkyle ou un groupe alcényle linéaire ou ramifié ayant 1 à 4 atomes de carbone et peuvent être identiques ou différents ; R³ représente un groupe alkyle ou un groupe alcényle linéaire ou ramifié ayant 1 à 22 atomes de carbone ; et X représente un groupe alkylène ou un groupe alcénylène linéaire ou ramifié ayant un à 10 atomes de carbone) et/ou un sel de ceux-ci.

2. Utilisation selon la revendication 1, dans laquelle le sel inorganique est choisi dans le groupe comprenant le chlorure de sodium et le chlorure de potassium.

3. Utilisation selon la revendication 1, dans laquelle le sel organique est choisi dans le groupe comprenant le glutamate de sodium (monohydraté), l'aspartate de sodium, l'aspartate de potassium, le lactate de sodium, le lactate de potassium, le malate de sodium, le malate de potassium et l'acétate de potassium.

4. Utilisation selon la revendication 1, dans laquelle l'agent tensioactif non ionique est choisi dans le groupe comprenant un ester d'acide gras et de sorbitan, un ester d'acide gras et de sorbitol polyoxyéthyléné, une huile de ricin polyoxyéthylénée durcie, un ester d'acide gras et de polyéthylène glycol, un ester pyroglutamate d'huile de ricin polyoxyéthylénée durcie et un ester pyroglutamate de glycéryle polyoxyéthyléné.

5. Utilisation selon la revendication 1, dans laquelle l'agent tensioactif ampholytique est choisi dans le groupe comprenant les agents tensioactifs ampholytiques de type carbobétaïne, les agents tensioactifs ampholytiques de type sulfobétaïne et les agents tensioactifs ampholytiques de type hydroxysulfobétaïne.

6. Utilisation selon l'une quelconque des revendications 1 à 5, dans laquelle R³ dans la formule générale (1) représente un groupe alkyle linéaire ayant 11 atomes de carbone.

7. Utilisation selon l'une quelconque des revendications 1 à 5, dans laquelle R¹ et R² dans la formule générale (1) représentent individuellement un atome d'hydrogène ; R³ représente un groupe alkyle linéaire ayant 11 atomes de carbone ; et X représente un groupe alkyle linéaire ayant 4 atomes de carbone.

8. Composition liquide contenant un dérivé guanidine contenant un groupe amide représenté par la formule générale (1) suivante: 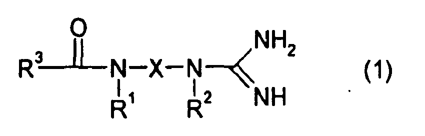 (dans la formule, R¹ et R² représentent indépendamment un atome d'hydrogène ou un groupe alkyle ou un groupe alcényle linéaire ou ramifié ayant un à quatre atomes de carbone et peuvent être identiques ou différents ; R³ représente un groupe alkyle ou un groupe alcényle linéaire ou ramifié ayant 1 à 22 atomes de carbone ; et X représente un groupe alkylène ou un groupe alcénylène linéaire ou ramifié ayant 1 à 10 atomes de carbone) et/ou un sel de ceux-ci (composant A) et au moins un composant B choisi parmi le chlorure de sodium et le chlorure de potassium, le glutamate de sodium (monohydraté), l'aspartate de sodium, l'aspartate de potassium, le lactate de sodium, le lactate de potassium, le malate de sodium, le malate de potassium et l'acétate de potassium, dans laquelle le rapport de mélange du composant A/composant B, en poids, est situé entre 1/0,5 et 1/20.

9. Composition liquide selon la revendication 8, dans laquelle R³ dans la formule générale (1) représente un groupe alkyle linéaire ayant 11 atomes de carbone.

10. Composition liquide selon la revendication 8, dans laquelle R¹ et R² dans la formule générale (1) représentent individuellement un atome d'hydrogène; R³ représente un groupe alkyle linéaire ayant 11 atomes de carbone ; et X représente un groupe alkyle linéaire ayant 4 atomes de carbone.

11. Matériau de base support imprégné tel qu'un tissu humide préparé par imprégnation à l'aide d'une composition liquide selon l'une quelconque des revendications 8 à 10.
